# FASCICULE DE BREVET EUROPEEN

(11) **EP 1 952 771 B1**
(45) Date de publication et mention de la délivrance du brevet: **12.10.2016**
(21) Numéro de dépôt: 08356017.7
(22) Date de dépôt: 30.01.2008
(51) Int. Cl.: A61B 17/16, A61B 17/17, A61F 2/30, A61F 2/40

(54) **Ensemble d'instrumentation chirurgicale pour poser une prothèse d'épaule**
Gesamtheit der chirurgischen Instrumente zum Einsetzen einer Schulterprothese
Surgical instrument assembly for inserting a shoulder prosthesis

(30) Priorité: 30.01.2007 FR 0700622; 06.02.2007 US 888437 P; 12.09.2007 US 971762 P
(43) Date de publication de la demande: 06.08.2008
(73) Titulaire: Tornier, 38330 Montbonnot-Saint-Martin (FR)
(72) Inventeur: Boileau, Pascal, 06200 Nice (FR); Walch, Gilles, 69003 Lyon (FR)
(74) Mandataire: Lavoix

(56) Documents cités:
- EP-A- 1 477 120
- EP-A- 1 570 816
- EP-A- 1 607 069
- WO-A-00/62718
- US-A1- 2005 245 934

## Description

La présente invention a trait, de manière générale, à la pose d'une prothèse d'épaule, en particulier d'une prothèse totale d'épaule inversée. Plus précisément, elle concerne un ensemble d'instrumentation chirurgicale pour poser une telle prothèse.

Dans le domaine des prothèses totales d'épaule, on parle couramment de prothèses inversées lorsque ces prothèses comportent, d'une part, une partie glénoïdienne solidaire de la glène d'une omoplate de l'épaule d'un patient et délimitant une surface articulaire convexe et, d'autre part, une partie humérale solidaire de l'humérus de l'épaule et délimitant une surface articulaire concave, la coopération de ces surfaces articulaires permettant de recréer une liaison articulée au niveau de l'épaule. Avec ce type de prothèse, il est fréquent, lors d'un mouvement d'adduction de l'épaule, que la portion inférieure de la partie prothétique humérale vienne heurter le pilier de l'omoplate, c'est-à-dire la partie inférieure de la glène osseuse située, lorsque le patient se tient debout, juste au-dessous de la partie prothétique glénoïdienne. Cette interférence entre la partie prothétique humérale et l'omoplate limite l'amplitude du mouvement d'adduction et peut induire des douleurs, pour le patient, voire conduire à un descellement de la prothèse, notamment par ostéolyse de l'omoplate.

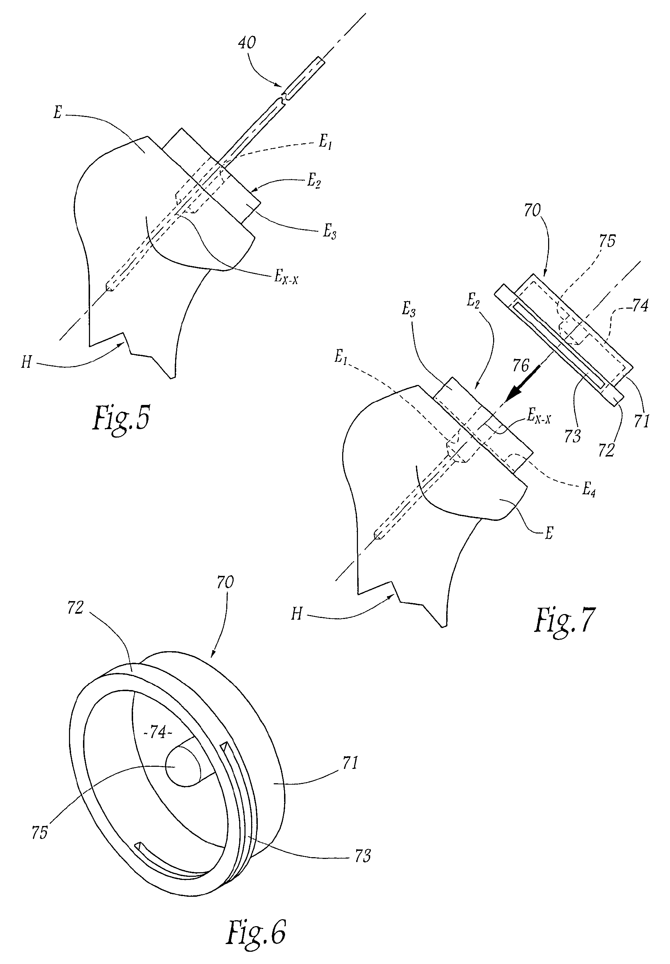

EP-A-1 477 120, qui peut être considéré comme l'état de la technique le plus proche de l'invention, divulgue un ensemble d'instrumentation chirurgicale pour poser une prothèse fémorale. La figure 6 de ce document montre un premier ancillaire permettant de conformer en un volume cylindrique la matière osseuse constituant l'épiphyse fémorale supérieure d'un fémur. La figure 7 montre une lame de résection de l'épiphyse fémorale, ainsi qu'un guide de coupe conçu pour ceinturer de manière complémentaire la totalité du volume cylindrique de matière osseuse obtenu par conformation de l'épiphyse au moyen du premier ancillaire précité : la lame de résection est prévue pour dégager du fémur uniquement l'extrémité de l'épiphyse qui émerge du guide de coupe, sans qu'il ne soit prévu que cette lame dégage le volume cylindrique de matière osseuse, étant remarqué que la hauteur du guide de coupe est telle que ce dernier s'étend, du côté tourné vers le fémur, au-delà de la base de ce volume cylindrique de matière osseuse, ce qui empêche toute action de la lame au niveau de cette base du volume cylindrique de matière osseuse. Le non-dégagement du volume cylindrique de matière osseuse est d'ailleurs cohérent avec le fait que la finalité de ce volume cylindrique de matière osseuse est de supporter une tête prothétique fémorale à l'intérieur de laquelle le volume cylindrique de manière osseuse est logé et impacté.

Le but de la présente invention est de proposer un ensemble d'instrumentation chirurgicale pour poser une prothèse d'épaule inversée, permettant de limiter les risques d'interférence entre l'omoplate et la partie humérale, éventuellement prothésée, de l'épaule, sans recourir à des aménagements complexes de la prothèse d'épaule, voire en utilisant pour l'essentiel des prothèses inversées existantes.

En pratique, l'invention trouve une application particulièrement avantageuse pour la pose des prothèses totales d'épaule inversées, mais s'applique également au domaine des prothèses d'épaule inversées « partielles », dont le composant glénoïdien coopère avec la tête épiphysaire anatomique d'un humérus, éventuellement resurfacée. L'invention peut également s'appliquer au domaine des prothèses d'épaule anatomiques, totales ou partielles.

A cet effet, l'invention a pour objet un ensemble d'instrumentation chirurgicale pour poser une prothèse totale d'épaule inversée, tel que défini à la revendication 1.

Pour bien comprendre l'intérêt de l'invention, on propose ici une méthode chirurgicale de pose d'une prothèse d'épaule, en particulier inversée, la prothèse incluant un composant glénoïdien qui présente une face articulaire, en particulier convexe, et une face opposée, cette méthode de pose comprenant des étapes peropératoires successives selon lesquelles :
i) on dispose d'un greffon osseux,
ii) on met en place le greffon sur la glène, préalablement préparée, d'une omoplate de l'épaule d'un patient, et
iii) on implante le composant glénoïdien, en recouvrant le greffon mis en place sur la glène par la face opposée du composant glénoïdien et en ancrant le composant glénoïdien dans la glène à travers le greffon.

Ainsi, l'idée à la base de cette méthode est de « latéraliser » le composant glénoïdien par rapport à l'omoplate du patient, c'est-à-dire de l'éloigner de l'omoplate du patient dans un plan frontal à ce patient, en interposant entre ce composant glénoïdien et la glène le greffon osseux. Autrement dit, ce greffon osseux forme, en quelque sorte, un prolongement latéral extérieur de la glène, s'éloignant de l'omoplate, tandis que l'association de ce greffon et du composant prothétique glénoïdien forme, en quelque sorte, un ensemble prothétique composite. On comprend qu'un composant glénoïdien d'une prothèse inversée actuelle, dont le dessin a fait ses preuves, peut ainsi être implanté, en recouvrant le côté du greffon opposé à la glène, étant remarqué que, à des fins de fixation sûre, ce composant doit présenter des moyens d'ancrage osseux, telle qu'une queue centrale, suffisamment allongée pour traverser de part en part le greffon et venir se fixer fermement dans l'os de l'omoplate délimitant la glène. Une fois que le greffon osseux a fusionné avec la glène, la surface distale du greffon osseux devient la surface glénoïdienne effective, c'est-à-dire fonctionnellement efficace. Dans ce contexte, les références à la glène sont à interpréter comme incluant la surface exposée, préparée ou non, d'une cavité glénoïdienne.

Comme la face articulaire du composant glénoïdien occupe, par rapport à l'omoplate, une position plus éloignée latéralement que la position que cette face occuperait en l'absence du greffon, les risques d'interférence entre le pilier de l'omoplate et la portion inférieure de la partie prothétique humérale coopérant avec la face articulaire glénoïdienne, sont significativement réduits. La latéralisation du composant prothétique glénoïdien induit également une augmentation de la tension des muscles rotateurs de l'épaule, ainsi qu'une augmentation du vecteur coapteur du muscle deltoïde. Les composants prothétiques glénoïdien et huméral s'en trouvent stabilisés et bénéficient ainsi d'une meilleurs mobilité en rotation relative, sans courir le risque d'une luxation de l'épaule. Bien entendu, l'intérêt principal lié à la structure dite inversée de la prothèse d'épaule est conservé, dans le sens où le centre géométrique d'articulation de la prothèse est située dans la glène : plus précisément, ce centre géométrique d'articulation est de préférence situé au niveau de la face osseuse dans la glène, étant remarqué que, dans le cas d'une prothèse d'épaule inversée, le rayon de courbure de la surface articulaire convexe du composant glénoïdien est avantageusement choisi pour que le centre de rotation soit dans ou derrière un plan comprenant la surface distale du greffon osseux.

Par ailleurs, par rapport à une prothèse d'épaule inversée relevant de l'art antérieur et que l'on peut donc qualifiée de « prothèse médialisée », la prothèse « latéralisée » selon l'invention redonne à l'épaule du patient un peu de son galbe, ce qui lui confère un aspect plus esthétique, comparé à l'aspect « portemanteau » conféré par les prothèses médialisées.

La méthode chirurgicale proposé ici est simple, rapide, facile et reproductible. En pratique, elle présente l'avantage de ne pas devoir exposer complètement la glène du patient, l'exposition pouvant en effet être limitée à la mise en place du greffon.

Selon une mise en oeuvre particulièrement avantageuse de cette méthode, pour disposer du greffon osseux à l'étape i), on prélève ce dernier dans l'épiphyse supérieure de l'humérus de l'épaule du patient. De la sorte, le greffon utilisé provient du patient, ce qui limite les risques de rejet, de mauvaise compatibilité biologique, de transmission de maladie ou d'infection. En outre, on met ainsi à profit le fait que, pour implanter la partie prothétique humérale, il est nécessaire de préparer l'épiphyse de l'humérus du patient, en retirant une partie non négligeable de la matière osseuse spongieuse constituant cette épiphyse, qui, selon cet aspect de l'invention, peut être utilisée pour disposer du greffon, alors que, jusqu'à maintenant, cette matière était mise au rebut.

En pratique, on met alors avantageusement en oeuvre :
- une étape de conformation, dans laquelle on conforme la matière osseuse constituant l'épiphyse humérale supérieure, en un volume monobloc qui s'étend en longueur autour d'un axe incliné par rapport à la direction longitudinale de l'humérus, et
- une étape de coupe, dans laquelle on dégage de l'humérus le volume de matière osseuse en coupant l'épiphyse humérale de manière transversale à l'axe de ce volume, le volume de matière osseuse ainsi dégagé constituant le greffon.

L'ensemble d'instrumentation selon l'invention permet de mettre en oeuvre la méthode de pose définie ci-dessus, dont les étapes de conformation et de coupe sont respectivement mises en oeuvre par les ancillaires de conformation et de coupe. Le volume de matière osseuse dégagé de l'humérus au moyen de l'ancillaire de coupe peut ainsi être utilisé comme greffon osseux pour la mise en oeuvre de la méthode de pose générale définie plus haut, en vue de décaler latéralement la face articulaire d'un composant glénoïdien de la prothèse par rapport à l'omoplate de l'épaule du patient, lors de l'implantation de ce composant glénoïdien.

Suivant d'autres caractéristiques avantageuses de la méthode de pose proposée ici :
- on ajuste, suivant l'axe du volume de matière osseuse, la longueur du greffon ;
- les faces d'extrémité longitudinale du greffon sont respectivement conformées pour être sensiblement complémentaires de ladite face opposée du composant glénoïdien et de la glène préalablement préparée ;
- lors de l'étape de conformation, le volume de matière osseuse conformé est choisi parmi un cylindre et un tronc de cône, centrés sur l'axe de ce volume ;
- avant ou au cours de l'étape de conformation, on résèque l'extrémité de l'épiphyse humérale supérieure ;
- l'épiphyse humérale supérieure est réséquée selon un premier plan et dans laquelle, lors de l'étape de coupe, l'épiphyse humérale est coupée selon un second plan, lesdits premier et second plans étant transversaux à l'axe du volume de matière osseuse ;
- on ajuste l'inclinaison relative desdits premier et second plans ;
- au cours de ou après l'étape de conformation, on creuse un évidement dans l'épiphyse humérale de manière centrée sur l'axe du volume de matière osseuse, et dans laquelle, lors de l'étape, on ancre le composant glénoïdien dans la glène à travers cet évidemment ;
- avant de mettre en oeuvre l'étape de conformation, on insère dans l'épiphyse humérale une broche de repérage permettant, lors de l'étape de conformation, de positionner l'axe du volume de matière osseuse par rapport à l'humérus.

Des caractéristiques avantageuses de l'ensemble d'instrumentation conforme à l'invention sont spécifiées aux revendications dépendantes 2 à 12, pour mettre en oeuvre les aspects de la méthode définis ci-dessus.

L'invention sera mieux comprise à la lecture de la description qui va suivre, donnée uniquement à titre d'exemple et faite en se référant aux dessins sur lesquels :
- la figure 1 est une représentation schématique de principe d'une prothèse totale d'épaule inversée, implantée au niveau de l'épaule d'un patient ;
- la figure 2 est une vue schématique en élévation d'un ancillaire d'un ensemble d'instrumentation selon l'invention, utilisé en vue de poser la prothèse de la figure 1 ;
- la figure 3 est une vue analogue à la figure 2, illustrant un autre ancillaire appartenant à l'ensemble d'instrumentation, à appliquer sur l'humérus du patient après l'utilisation de l'ancillaire de la figure 2 ;
- la figure 4 est une vue schématique en perspective d'encore un autre ancillaire appartenant à l'ensemble d'instrumentation ;
- la figure 5 est une vue analogue à la figure 2, illustrant l'humérus après l'utilisation des ancillaires des figures 3 et 4 ;
- la figure 6 est une vue schématique en perspective d'un autre ancillaire appartenant à l'ensemble d'instrumentation ;
- la figure 7 est une vue analogue à la figure 2, illustrant l'application de l'ancillaire de la figure 6 sur l'humérus après l'utilisation de l'ancillaire de la figure 4 ;
- les figures 8 à 12 illustrent un second mode de réalisation d'un ensemble d'instrumentation conforme à l'invention, les figures 8, 9, 11 et 12 étant respectivement des vues schématiques en élévation analogues de quatre ancillaires appartenant à cet ensemble et successivement utilisés, en vue de poser la prothèse de la figure 1, tandis que la figure 10 est une vue partielle en perspective de l'ancillaire de la figure 9, représenté seul ; et
- la figure 13 est une représentation schématique de principe de la partie glénoïdienne d'une autre prothèse totale d'épaule inversée.

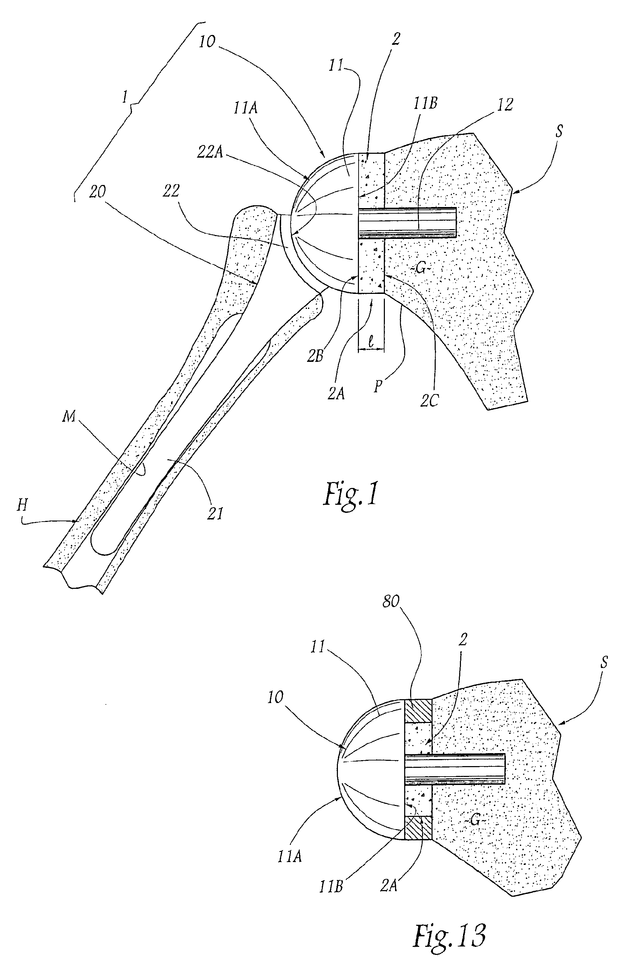

Sur la figure 1 est représentée une prothèse d'épaule 1, comprenant un composant glénoïdien 10 et un composant huméral 20, respectivement implantés dans l'omoplate S et l'humérus H de l'épaule d'un patient.

Le composant glénoïdien 10 comprend une tête 11 qui présente, du côté opposé à la glène G de l'omoplate S, une face articulaire convexe 11A de forme globalement hémisphérique et, du côté tourné vers la glène, une face opposée 11 B. Dans l'exemple considéré aux figures, cette face 11 B est globalement plane, mais, en variantes non représentées, cette face 11 B peut présenter une géométrie plus élaborée, en étant par exemple essentiellement concave ou convexe.

Le composant glénoïdien 10 comprend également une queue d'ancrage 12 qui s'étend transversalement en saillie de la face 11 B, en direction opposée à la face 11 A, et dont la partie d'extrémité libre est fermement ancrée dans la glène G, assurant ainsi la solidarisation du composant glénoïdien à l'omoplate S. En pratique, de manière non représentée, la queue d'ancrage 12 peut être pourvue, à son extrémité tournée vers la tête 11, d'une embase logée à l'intérieur de la tête 11, en étant fermement solidarisée à celle-ci. Autrement dit, de manière plus générale, la liaison entre la queue 12 et la tête 11 peut prendre des formes très diverses, telles qu'une continuité de matière, des surfaces respectives de coincement, des moyens rapportés d'assemblage mécanique, etc. Egalement à titre de variante non représentée, la queue 12 peut être extérieurement filetée ou, de manière générale, présenter un état de surface favorisant son ancrage.

Entre la face 11 B de la tête glénoïdienne 11 et la glène G de l'omoplate S est interposé un greffon osseux 2 présentant une forme extérieure sensiblement cylindrique à base circulaire, dont le diamètre extérieur est sensiblement égal à celui de la tête 11. Ainsi, la face latérale extérieure 2A du greffon 2 s'étend sensiblement dans le prolongement de la face hémisphérique 11A. Le greffon 2 présente, sur son côté opposé à la glène G, une face d'extrémité longitudinale 2B recouverte par la face 11 B de la tête 11 et, sur son côté dirigé vers la glène, une face d'extrémité longitudinale 2C en appui contre la glène G. Dans l'exemple considéré aux figures, les faces d'extrémité longitudinale 2B et 2C sont planes, ce qui s'avère être une forme de réalisation facile à manipuler et simple à obtenir, comme évoqué plus loin. Toutefois, en pratique, ces faces 2B et 2C peuvent présenter des géométries plus élaborées : d'un côté, la face 2B est prévue pour être recouverte de manière sensiblement complémentaire par la face 11 B de la tête 11, en incluant dans cette face les zones ou les moyens de liaison avec la queue 12, étant entendu que, comme indiqué plus haut, cette face 11 B peut être globalement concave, convexe ou plane ; du côté opposé, la face 2C est prévue pour épouser la surface de la glène G, qui a été préalablement préparée à cet effet, de sorte que la face 2C et la glène sont sensiblement complémentaires, en pouvant être aussi bien planes que courbes.

Le greffon 2 est traversé de part en part, suivant sa direction longitudinale, par la queue 12. Autrement dit, cette dernière présente une longueur bien supérieure à celle du greffon 2, de manière qu'au moins une partie non négligeable de cette queue s'ancre fermement dans la couche native de la glène G.

A titre d'aménagements optionnels non représentés, la fixation du greffon à la glène peut être renforcée par des moyens additionnels à la queue 12, tels que des vis réparties autour de cette queue et traversant le greffon au moins sur une partie de sa longueur.

Le composant huméral 20 comprend une queue 21 d'ancrage dans le canal médullaire M de l'humérus H. A son extrémité supérieure, cette queue est pourvue d'une tête 22 qui présente, sur son côté opposé à la queue 21, une face articulaire concave 22A en forme de tronçon de sphère, de rayon sensiblement égal à celui de la face 11A. Lorsque la prothèse 1 est implantée, comme sur la figure 1, les faces 11A et 22A sont en appui surfacique l'une contre l'autre, ce qui permet les différents mouvements articulaires d'épaule recherchés.

Compte tenu de la présence du greffon 2, la face 11A se trouve éloignée de la surface réséquée de la glène G, dans le sens où, en l'absence de ce greffon, cette face 11A serait directement juxtaposée à la surface réséquée de la glène. Ainsi, grâce au greffon 2, la face articulaire glénoïdienne 11A et, par conséquent, la face articulaire humérale 22A, sont éloignées latéralement de la glène G, limitant les risques que la portion inférieure de la tête 22 vienne interférer avec la partie basse de la glène G, c'est-à-dire avec le pilier P de l'omoplate S. En outre, on comprend que, à titre de conséquence induite à cette latéralisation recherchée dans le cadre de l'invention, le greffon 2 fait office de matière osseuse pour combler un éventuel déficit d'os au niveau de la glène.

En pratique, le composant glénoïdien 10 peut présenter des tailles diverses, auxquelles le greffon 2 est adapté. Typiquement, la tête 11 est disponible en au moins deux tailles différentes, à savoir avec un diamètre extérieur valant 36 mm ou 42 mm, étant entendu que d'autres tailles sont envisageables. De même, la longueur l du greffon 2 peut présenter des valeurs différentes, réparties en pratique en une série de pas constant, de manière adaptée à la morphologie et/ou à la pathologie du patient. Le greffon 2 peut ainsi présenter des longueurs de 3, 6, 8 ou 10 mm, tandis que la queue 12 présente une longueur comprise entre 15 et 25 mm, voire davantage.

On va décrire ci-après une méthode chirurgicale visant à implanter la prothèse d'épaule 1 de la figure 1, étant entendu que la prothèse considérée n'est qu'un exemple illustratif non limitatif de la méthode et des instruments chirurgicaux utilisés pour implanter cette prothèse. Autrement dit, la méthode et les instruments détaillés ci-après peuvent être utilisés pour implanter des prothèses d'épaule de structures diverses, par exemple dont les composants glénoïdiens et/ou huméraux sont constitués de plusieurs parties assemblées les unes aux autres, de nature métallique, plastique et/ou céramique. Ainsi, l'utilisation d'un composant huméral dépourvu de queue d'ancrage est par exemple possible. De surcroît, des prothèses d'épaule inversées « partielles » peuvent également être considérées, c'est-à-dire des prothèses qui sont dépourvues de composant huméral et dont le composant glénoïdien est prévu pour, en service coopérer de manière articulée avec la tête épiphysaire anatomique du fémur, éventuellement resurfacée au préalable.

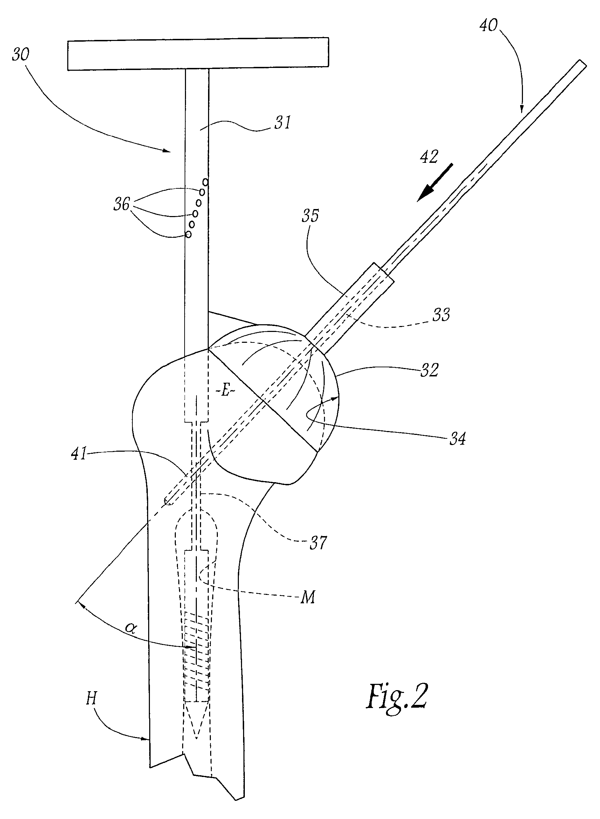

Dans un premier temps opératoire, après avoir dégagé les parties molles de l'épaule suivant une approche delto-pectorale ou supéro-externe, on introduit dans le canal médullaire M de l'humérus H le manche 31 d'un ancillaire 30, en traversant de part en part l'épiphyse supérieure E de l'humérus H, comme représenté sur la figure 2. Pour ce faire, le point d'entrée dans l'épiphyse humérale est préalablement déterminé par analyse de radiographies de face et de profil de l'humérus.

Dans sa partie courante le manche 31 est lié, notamment de façon amovible, à un corps 32 en forme de cloche bombée vers le haut. Ce corps 32 est agencé globalement de manière transversale au manche 31, en s'étendant en longueur autour d'un axe géométrique central 33. Projeté dans un plan médio-latéral au patient contenant l'axe longitudinal du manche 31, comme vu à la figure 2, cet axe 33 est incliné par rapport à l'axe longitudinal du manche sous un angle α compris entre 10 et 70°, étant remarqué que, dans l'espace, les deux axes précités ne sont pas nécessairement sécants, mais se croisent de manière légèrement distante l'un de l'autre suivant une direction antéropostérieure.

Le corps 32 présente intérieurement une surface concave 34 dont le centre principal de courbure et le sommet appartiennent sensiblement à l'axe 33. Cette surface 34 est prévue pour reproduire approximativement les caractéristiques de surface de l'épiphyse supérieure d'un humérus anatomique normal, étant entendu que, en pratique, le chirurgien dispose d'une gamme de plusieurs ancillaires 30 homotétiques, dont les corps 32 présentent des dimensions respectives associées à la taille et à l'état des os du patient. Sur sa face extérieure, le corps 32 est pourvu d'un tube saillant 35 centré sur l'axe 33 et débouchant à l'intérieur du corps 32, sur sa surface intérieure 34.

Le manche 31 est inséré dans le canal médullaire M de l'humérus H jusqu'au contact entre la surface 34 et l'épiphyse humérale E, le corps 32 recouvrant alors l'épiphyse à la façon d'un calot. Puis, avantageusement, le manche 31 est entraîné en rotation sur lui-même, sur une faible course, pour tenir compte de la rétroversion de l'humérus H : de manière connue en soi, le manche 31 est pourvu, dans sa partie d'extrémité proximale, d'orifices diamétraux traversants 36 décalés angulairement les uns par rapport aux autres autour de l'axe longitudinal du manche 31 et, en fonction de la rétroversion du patient déterminée par le chirurgien, une tige allongée, non représentée, est introduite dans l'un de ces orifices pour bien visualiser la direction de rétroversion retenue, de manière que le manche 31 est tourné sur lui-même jusqu'à l'alignement de cette tige de rétroversion avec l'avant-bras du patient.

Une broche 40, à l'extrémité distale 41 pointue, est ensuite introduite dans le tube 35, depuis son extrémité libre, et est insérée dans l'épiphyse humérale E sur une profondeur substantielle, comme indiqué par la flèche 42 à la figure 2, jusqu'à ce que sa pointe vienne se piquer et traverser au moins en partie la corticale externe de l'humérus H. On comprend que l'ancillaire 30 permet d'insérer la broche suivant une direction ajustée par rapport à l'humérus, le tube 35 servant de guide d'introduction et de progression pour la broche. Pour éviter une interférence entre la broche et le manche 31, lorsque cette broche traverse la zone centrale de l'humérus, la partie courante correspondante 37 du manche 31 est avantageusement amincie : ainsi, cette partie de manche 37 présente une section transversale plus petite que les parties d'extrémité proximale et distale constituant le reste du manche.

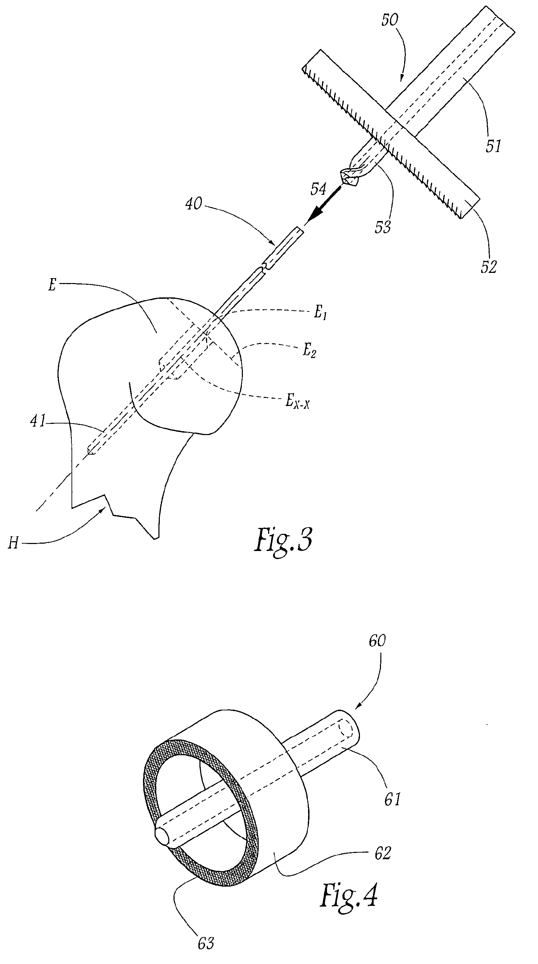

Une fois que la broche 40 a atteint une profondeur d'insertion dans, voire au travers de l'humérus H suffisante pour assurer fermement son ancrage, l'ancillaire 30 est retiré, sans enlever la broche. L'humérus est alors dans l'état représenté en traits pleins à la figure 3.

En variante, à la mise en oeuvre du premier temps opératoire, la broche 40 est insérée dans l'humérus H sans être guidée, c'est-à-dire sans utiliser l'ancillaire 30.

Dans un second temps, le chirurgien va réséquer l'extrémité de l'épiphyse humérale E, au moyen d'un ancillaire 50 représenté à la figure 3. Cet ancillaire 50 comporte un corps tubulaire 51 dont l'alésage central interne présente un diamètre égal au diamètre extérieur de la broche 40. A l'extrémité distale de ce corps 51, l'ancillaire 50 comporte une fraise plate 52, s'étendant dans un plan sensiblement perpendiculaire à l'axe longitudinal du corps 51. En saillie distale de cette fraise 52 et de manière centrée sur l'axe longitudinal du corps 51, l'ancillaire 50 comporte en outre un foret terminal 53 délimitant intérieurement un alésage central communiquant avec l'alésage du corps 51. Le diamètre extérieur du foret 53 est prévu égal au diamètre extérieur de la queue d'ancrage 12 du composant glénoïdien 10 à implanter, pour des raisons qui apparaîtront plus loin.

Le chirurgien enfile l'ancillaire 50 autour de la broche 40, en l'introduisant par son foret terminal 53, comme indiqué par la flèche 54 sur la figure 3. Lorsque ce foret atteint l'extrémité de l'épiphyse E, il fore la matière osseuse, de manière à former un évidement cylindrique E₁ centré autour de la broche 40, indiqué en pointillés à la figure 3. De même, au fur et à mesure que l'ancillaire 50 progresse vers le bas le long de la broche 40, la fraise 52 résèque progressivement l'extrémité de l'épiphyse humérale E, et ce sur une épaisseur de quelques millimètres, jusqu'à obtenir un plan de coupe E₂ perpendiculaire à la broche 40, indiqué également en pointillés à la figure 3.

Dans un troisième temps, après avoir dégagé l'ancillaire 50 de la broche 40, le chirurgien va « circoncire » l'épiphyse humérale E de manière centrée sur la broche 40, c'est-à-dire qu'il va conformer la matière osseuse constituant cette épiphyse en un cylindre E₃ d'axe central E_{X-X} correspondant à l'axe 33, comme représenté à la figure 5. A cet effet, le chirurgien utilise un ancillaire 60 représenté à la figure 4. Cet ancillaire 60 comporte une tige centrale 61, alésée intérieurement de manière complémentaire à la broche 40 et présentant un diamètre extérieur égal à celui du foret 53. Cette tige 61 porte, dans sa partie courante, une scie-cloche 62, de forme annulaire centrée sur la tige 61 et dont le chant d'extrémité distale présente une denture 63.

La tige 61 de l'ancillaire 60 est enfilée autour de la broche 40 laissée en place dans l'épiphyse humérale E, jusqu'à ce que son extrémité distale soit reçue de manière complémentaire dans l'évidement E₁. Ce faisant, la scie 62 découpe progressivement la matière osseuse de l'épiphyse, de manière à obtenir le cylindre osseux E₃, étant remarqué que ce dernier est traversé sur toute sa longueur par une partie correspondante de l'évidement E₁. La longueur du cylindre E₃ ainsi obtenu, c'est-à-dire sa dimension suivant son axe E_{X-X}, est déterminée par la profondeur d'action de la scie 62, cette profondeur pouvant être facilement repérée le long de la tige 61, notamment par des graduations.

Après avoir dégagé l'ancillaire 60, l'humérus H est dans l'état représenté à la figure 5.

Dans un quatrième temps, le chirurgien va dégager de l'humérus H le cylindre de matière osseuse E₃, en utilisant un ancillaire de coupe 70 représenté sur les figures 6 et 7. Cet ancillaire comporte un bloc tubulaire 71 dont le diamètre intérieur est égal à celui de la scie 62. A son extrémité distale, le bloc 71 forme un bord saillant extérieur 72, dans lequel est délimitée une fente transversale 73 débouchant dans le volume interne du bloc. A son extrémité proximale, le bloc 71 est fermé par une paroi de fond 74, depuis la zone centrale de laquelle s'étend en saillie, à l'intérieur du bloc, un téton de centrage 75 dont le diamètre extérieur est égal à celui du foret 53.

Après avoir dégagé la broche 40, l'ancillaire 70 est enfilée autour du cylindre huméral E₃, comme indiqué par la flèche 76 à la figure 7. Le cylindre E₃ est reçu de manière complémentaire dans le bloc 71, jusqu'à ce que le bord 72 vienne s'appuyer contre la surface osseuse entourant la base du cylindre E₃. Une lame de scie plane, non représentée, est alors introduite depuis l'extérieur dans la fente 73, pour couper la base du cylindre E₃ selon un plan de coupe E₄, sensiblement perpendiculaire à l'axe E_{X-X} et indiqué en pointillés à la figure 7. Durant le sciage, l'essentiel du cylindre E₃ est protégé par le bloc 71 et la paroi de fond 74, étant remarqué que le téton 75 est logé de manière complémentaire dans la partie d'extrémité supérieure de l'évidement central E₁.

Après dégagement de l'ancillaire 70, le chirurgien récupère le cylindre de matière osseuse E₃ ainsi séparé de l'humérus H.

En variante non représentée, la fente 73 peut être prévue inclinée par rapport à la direction longitudinale du bloc 71, de sorte que, à la différence du cylindre E₃ décrit ci-dessus, le cylindre osseux alors obtenu présente des faces d'extrémité longitudinale inclinées l'une par rapport à l'autre. De la sorte, le greffon peut rattraper l'usure d'une portion périphérique de la glène G, étant remarqué que l'inclinaison de la fente 73 est avantageusement réglable en fonction de l'usure constatée par le chirurgien, au cours de l'intervention.

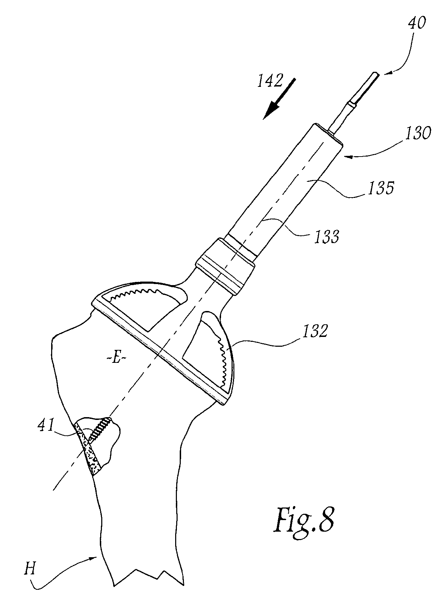

Avant de passer à la description du temps opératoire suivant, à savoir le cinquième temps, on considère ci-après les figures 8 à 12 qui illustrent un ensemble d'instrumentation constituant une variante de l'ensemble comprenant les ancillaires 30, 50, 60 et 70 décrits jusqu'ici.

Ainsi, en variante de l'ancillaire 30 de la figure 2, un ancillaire 130 est représenté à la figure 8. Cet ancillaire 130 comporte un corps distal 132 fonctionnellement analogue au corps 32 de l'ancillaire 30. En particulier, le corps 132 est dimensionné pour recouvrir l'épiphyse humérale supérieure E à la façon d'un calot. A la différence du corps 32 de l'ancillaire 30, le corps 132 est ajouré, notamment pour donner au chirurgien une meilleure visibilité de l'épiphyse humérale lors de la mise en place du corps 132. Comme le corps 32 de l'ancillaire 30, le corps 132 est pourvu, en saillie de sa face extérieure, d'un tube proximal 135 centré sur l'axe 133 autour duquel s'étend le corps 132.

L'ancillaire 130 permet d'insérer la broche 40 dans l'épiphyse humérale E, de manière ajustée par rapport à l'humérus H, comme indiqué par la flèche 142 à la figure 8.

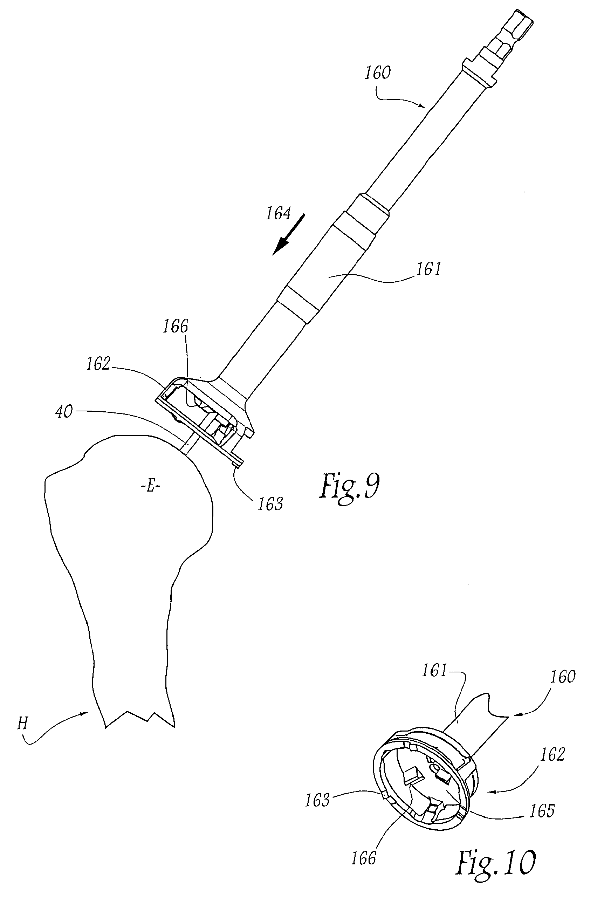

En variante à, à la fois, l'ancillaire 50 et l'ancillaire 60 des figures 3 et 4, un ancillaire 160 est représenté aux figures 9 et 10. Cet ancillaire 160 comporte un manche allongé 161 pourvu, à son extrémité distale, d'une scie-cloche 162, de forme annulaire centrée sur le manche 161 et dont le chant d'extrémité distale présente une denture 163. Le manche 161 est alésé intérieurement sur toute sa longueur, de manière à pouvoir être enfilé, de manière ajustée et coaxiale, autour de la broche 40 laissée en place dans l'épiphyse humérale E, comme indiqué par la flèche 164 à la figure 9. A la différence de la scie 62 de l'ancillaire 60, la scie 162 présente des ajours dans sa paroi latérale et comporte une paroi de fond 165, qui s'étend perpendiculairement à la direction longitudinale du manche 161 et dont la face distale forme une fraise plate 166.

De la sorte, lorsque l'ancillaire 160 est enfilé autour de la broche 40, la denture 163 de la scie 162 découpe progressivement la matière osseuse de l'épiphyse humérale E, de manière à obtenir le cylindre osseux E₃. Une fois que toute la hauteur de la scie 162 est ainsi introduite dans l'épiphyse, la fraise 166 entame l'extrémité supérieure de cet épiphyse, et résèque ainsi progressivement cette extrémité, jusqu'à obtenir le plan de coupe E₂.

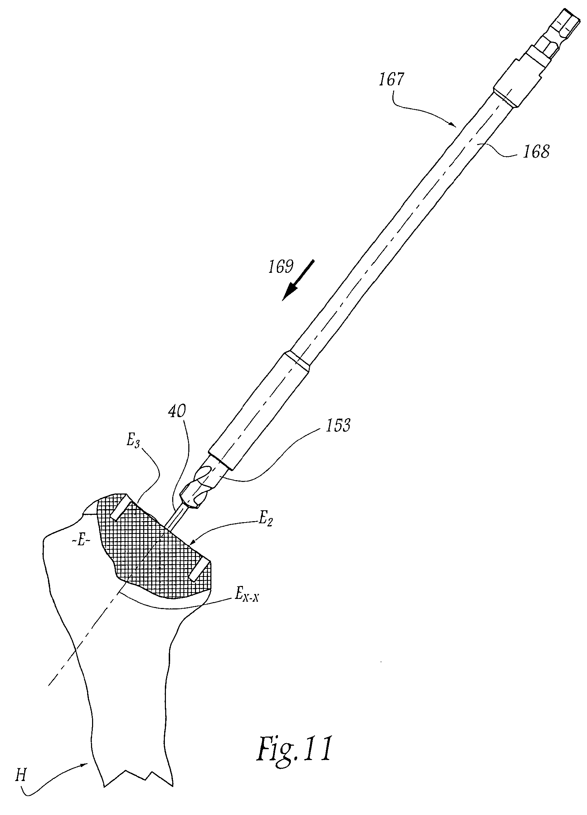

Après avoir dégagé l'ancillaire 160, l'humérus H est dans l'état représenté à la figure 11.

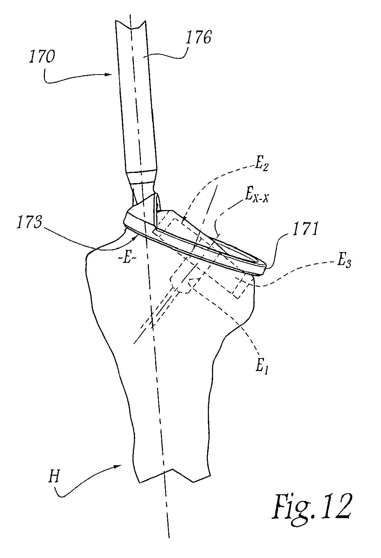

Le chirurgien utilise ensuite un ancillaire de forage 167 comportant un manche alésé 168 dont l'extrémité distale est pourvue d'un foret 153. En enfilant le manche 168 autour de la broche 40, comme indiqué par la flèche 169 à la figure 11, le chirurgien creuse, par l'action du foret 153, la partie centrale du cylindre de matière osseuse E₃ autour de la broche 40, de manière à former l'évidement E₁ de manière centrée sur l'axe E_{X-X} du cylindre E₃, comme indiqué en pointillés à la figure 12 sur laquelle l'ancillaire 168 a été dégagé.

En pratique, l'ancillaire de forage 167 peut par ailleurs être utilisé après avoir utilisé une variante de l'ancillaire 50, dépourvue du foret 53, et/ou après avoir utilisé une variante de l'ancillaire 60, dont la tige 61 ne s'étend pas en saillie du côté distal de la paroi de fond de la scie 62.

En variante à l'ancillaire 70 des figures 6 et 7, un ancillaire 170 est représenté à la figure 12. Cet ancillaire 170 comporte un corps annulaire 171 muni, en un point de sa périphérie, d'un manche proximal de manipulation 176. Le corps annulaire 171 est dimensionné pour être rapporté sur l'épiphyse humérale E en entourant toute la partie de l'épiphyse dans laquelle est délimité le cylindre de matière osseuse E₃ préalablement découpé par l'ancillaire 160. Sur son côté distal, le corps 171 délimite une surface 173 d'application et de guidage d'un outil de coupe osseuse, non représenté, tel qu'une lame de scie ou analogue.

De la sorte, en manipulant le manche 176, le chirurgien positionne le corps annulaire 171 autour de l'épiphyse humérale E, de manière à positionner la surface de guidage 173 de manière adéquate par rapport au cylindre de matière osseuse E₃. En appliquant ensuite l'outil de coupe de manière guidée contre cette surface 173, le chirurgien coupe la base du cylindre E₃ selon la plan de coupe E₄, afin de dégager ce cylindre de l'humérus H.

Avantageusement, la surface de guidage 173 forme avec la direction longitudinale du manche 176 un angle d'environ 155°, ce qui permet d'utiliser également l'ancillaire 170 pour préparer ultérieurement l'implantation du composant huméral 20, en positionnant le manche 176 de sorte que sa direction longitudinale soit sensiblement alignée avec la direction longitudinale de l'humérus H, comme illustré à la figure 12.

Dans un cinquième temps, le cylindre de matière osseuse E₃ est utilisé pour constituer le greffon osseux 2 décrit plus haut. Pour ce faire, ce cylindre est mis en place sur la glène G. Cette dernière est à cet effet préalablement préparée, en étant avivée et, si besoin, réséquée. Le composant glénoïdien 10 est ensuite implanté dans la configuration décrite ci-dessus en regard de la figure 1. On comprend que la queue d'ancrage 12 est introduite co-axialement dans l'évidement central E₁ du cylindre E₃, de manière sensiblement ajustée.

Si les faces d'extrémité longitudinales du cylindre osseux ont été réalisées de manière inclinée l'une par rapport à l'autre, on comprend que l'interposition de ce cylindre, en tant que greffon, entre le composant glénoïdien 10 et la glène G permet d'incliner, notamment vers le bas, la face articulaire glénoïdienne 11 A.

De manière plus générale, on comprend que les dimensions voulues par le chirurgien pour le greffon 2, notamment en fonction de la taille du composant glénoïdien 10, déterminent les dimensions des ancillaires 50, 60 et 70 ou des ancillaires 160, 168 et 170 utilisés pour prélever le cylindre osseux E₃ depuis l'épiphyse humérale E. En particulier, le diamètre intérieur de la scie 62 ou 162 détermine le diamètre extérieur du greffon 2. De même, la profondeur d'action de cette scie détermine la longueur l du greffon, tout en tenant compte d'un éventuel ajustement en longueur par le positionnement de la fente de sciage 73 ou de la surface de guidage 173.

En outre, la géométrie voulue pour les faces d'extrémité longitudinale 2B et 2C du greffon 2 conditionne directement la forme de réalisation des ancillaires de résection 50 et de coupe 70 ou des ancillaires 160 et 170, dans le sens où les parties de ces ancillaires qui déterminent le profil d'entame de l'os sont conformées pour entamer l'épiphyse humérale de manière adéquate. De manière optionnelle, ces ancillaires 50 et 70 peuvent être associés à un ou plusieurs ancillaires de resurfaçage des faces d'extrémité longitudinale du cylindre prélevé E₃.

En pratique, le chirurgien tient également compte de l'état de la matière osseuse spongieuse constituant l'épiphyse E, pour, si nécessaire, prélever le greffon avec une constitution la plus saine possible. A ce propos, de manière optionnelle, des ancillaire de préhension et de stockage du greffon 2 après qu'il ait été dégagé de l'humérus H peuvent être prévus, afin de limiter les risques d'endommagement du greffon.

En outre, à titre de variantes non représentées, le greffon 2 peut présenter d'autres formes volumiques qu'un cylindre comme sur les figures, du moment que le volume de matière osseuse constituant ce greffon présente une forme globalement centrée autour d'un axe longitudinal du type de l'axe E_{X-X}, tout en définissant une face latérale et des faces d'extrémité longitudinale du type des faces 2A, 2B et 2C. A titre d'exemple, le greffon peut ainsi être de forme tronconique, d'axe longitudinal E_{X-X} ; dans ce cas, la surface intérieure de la scie-cloche 62 ou 162 est par exemple prévue tronconique.

De manière optionnelle, le greffon osseux 2 peut être protégé latéralement, de préférence par un anneau 80 représenté uniquement à la figure 13. En pratique, cet anneau est dimensionné pour entourer de manière ajustée la face latérale 2A du greffon 2, sur toute la longueur de ce greffon. L'anneau 80 se trouve ainsi, conjointement au greffon 2, interposé entre le composant glénoïdien 10 et la glène G. On comprend que cet anneau peut, par exemple, être utilisé lorsque le greffon présente, au moins sur une partie de sa longueur, un diamètre extérieur inférieur à celui de la tête glénoïdienne 11, l'anneau permettant ainsi de rattraper la différence de diamètre.

Lorsque l'anneau 80 est implanté conjointement avec le greffon 2, il protège la face latérale 2A du greffon et forme un appui pour au moins une partie de la face 11B du composant glénoïdien 10, limitant ainsi les contraintes appliquées au greffon. Avantageusement, l'anneau 80 est recouvert d'hydroxyapatite ou, plus généralement, présente un état de surface poreux ou en nid d'abeilles, permettant d'améliorer l'adhérence et la réhabilitation osseuse de l'anneau au greffon et à la partie de surface réséquée de la glène G qui n'est pas recouverte par ce greffon.

En pratique, on comprend que la surface interne de l'anneau 80 est avantageusement complémentaire de la face 2A du greffon, tandis que sa surface externe peut présenter des aménagements optionnels avantageux. Ainsi, cette surface externe peut être prévue tronconique divergente vers la glène G, de manière que des trous traversant l'anneau suivant des directions respectives sensiblement perpendiculaires à sa surface externe, puissent recevoir des vis ou analogues pour renforcer la fixation du greffon à la glène. De même, la portion basse de l'anneau 80 peut être prévue moins épaisse que le reste de l'anneau, pour ne pas gêner ultérieurement le composant huméral 20 lors de mouvements d'adduction du patient.

Avant ou après l'implantation du composant glénoïdien 10, le composant huméral 20 est implanté dans l'humérus H, en utilisant avantageusement des ancillaires, non représentés, dont les manipulations sont repérées par la partie d'extrémité de l'évidement E₁ subsistant dans l'épiphyse humérale E après dégagement du volume osseux tel que le cylindre E₃. Si on renonce à repérer les gestes chirurgicaux appliqués à l'humérus pour implanter le composant 20 grâce à l'évidement E₁ et que ces gestes sont alors globalement indépendants de ceux appliqués à l'humérus pour prélever le greffon 2, l'ancillaire 30 peut être simplifié puisqu'il n'est alors plus nécessaire de tenir compte de la rétroversion de l'avant-bras du patient pour insérer la broche 40. Le manche 31 peut alors prendre la forme d'une tige humérale intra-médullaire.

## Revendications

1. Ensemble d'instrumentation chirurgicale pour poser une prothèse d'épaule inversée (1), qui comporte :
- un ancillaire (60 ; 160) de conformation de la matière osseuse constituant l'épiphyse humérale supérieure (E) d'un humérus (H), en un volume monobloc (E₃) qui s'étend en longueur autour d'un axe (E_{X-X}) incliné par rapport à la direction longitudinale de l'humérus (H) ; et
- un ancillaire (70 ; 170) de coupe de l'épiphyse humérale (E) conformée par l'ancillaire de conformation (60 ; 160), pour couper le volume de matière osseuse (E₃) de manière transversale à l'axe (E_{X-X}) de ce volume, cet ancillaire de coupe comportant :
(a) un bloc tubulaire (71) adapté pour être enfilé autour du volume de matière osseuse (E₃) conformé par l'ancillaire de conformation (60), ce bloc tubulaire délimitant, à son extrémité longitudinale tournée en service vers l'humérus (H), une fente transversale (73) de passage d'une lame de scie ou analogue, pour couper le volume de matière osseuse (E₃) selon un premier plan (E₄) transversal à l'axe (E_{X-X}) de ce volume, ou
(b) un corps annulaire (171) adapté pour être rapporté autour de l'épiphyse humérale (E) en entourant, à la fois, le volume de matière osseuse (E₃) conformé par l'ancillaire de conformation (160) et au moins une partie du reste de l'épiphyse humérale, le corps annulaire délimitant, sur son côté tourné en service vers l'humérus (H), une surface de guidage (173) d'un outil de coupe osseuse, pour couper au moins le volume de matière osseuse (E₃) de manière transversale à son axe (E_{X-X}), ce bloc tubulaire (71) et ce corps annulaire (171) permettant ainsi de dégager de l'humérus (H) le volume de matière osseuse (E₃) pour que ce dernier constitue un greffon (2) de latéralisation d'un composant glénoïdien (10) de la prothèse d'épaule inversée (1) par rapport à l'omoplate (S) d'un patient à prothéser.

2. Ensemble suivant la revendication 1, **caractérisé en ce qu'**il comprend des moyens (52 ; 166) de résection de l'extrémité de l'épiphyse humérale (E), qui sont soit portés par un ancillaire de résection spécifique (50), distinct des autres ancillaire (60, 70) de l'ensemble, soit intégrés à l'ancillaire de conformation (160).

3. Ensemble suivant la revendication 2, **caractérisé en ce que** les moyens de résection comporte une fraise plate (52 ; 166), de manière à réséquer l'épiphyse humérale (E) selon un second plan (E₂) transversal à l'axe (E_{X-X}) du volume de matière osseuse (E₃).

4. Ensemble suivant l'une quelconque des revendications précédentes, **caractérisé en ce qu'**il comprend des moyens (53 ; 153) de forage de l'épiphyse humérale (E), qui sont adaptés pour creuser un évidement (E₁) dans l'épiphyse humérale de manière centrée sur l'axe (E_{X-X}) du volume de matière osseuse (E₃) et qui sont soit intégrés à l'ancillaire de conformation ou à l'ancillaire de résection (50), soit portés par un ancillaire de forage spécifique (167), distinct des autres ancillaires (130, 160, 170) de l'ensemble.

5. Ensemble suivant l'une quelconque des revendications précédentes, **caractérisé en ce qu'**il comporte en outre une broche de repérage (40) ou un moyen analogue, à même d'être inséré dans l'épiphyse humérale (E) et adapté pour guider l'ancillaire de conformation (60 ; 160) et éventuellement au moins un des autres ancillaires (50 ; 167) de l'ensemble.

6. Ensemble suivant la revendication 5, **caractérisé en ce qu'**il comporte en outre un ancillaire (30 ; 130) d'insertion de la broche de repérage (40) dans l'épiphyse humérale (E), adapté pour ajuster la direction d'insertion de cette broche par rapport à l'humérus (H).

7. Ensemble suivant la revendication 6, **caractérisé en ce que** l'ancillaire d'insertion (30 ; 130) comporte, d'une part, un corps (32 ; 132) en forme de cloche bombée, dimensionnée intérieurement pour couvrir l'épiphyse humérale supérieure (E) à la façon d'un calot et, d'autre part, un guide (35) d'application de la broche de repérage, qui débouche dans le corps.

8. Ensemble suivant l'une quelconque des revendications précédentes, **caractérisé en ce que** l'ancillaire de conformation (60 ; 160) comporte une scie (62 ; 162) en forme de cloche, qui est adaptée pour couper la matière osseuse constituant l'épiphyse humérale (E) en la conformant en ledit volume de matière osseuse (E₃).

9. Ensemble suivant la revendication 8, **caractérisé en ce que** la scie (62 ; 162) présente une face intérieure cylindrique ou tronconique, éventuellement ajourée, pour donner au volume de matière osseuse une forme globale de cylindre (E₃) ou de tronc de cône, centré sur l'axe (E_{X-X}) de ce volume.

10. Ensemble suivant l'une quelconque des revendications précédentes, **caractérisé en ce que** l'ancillaire de coupe (70) comporte le bloc tubulaire (71), à l'exclusion du corps annulaire (171).

11. Ensemble suivant l'une quelconque des revendications 1 à 9, **caractérisé en ce que** l'ancillaire de coupe (170) comporte le corps annulaire (171), à l'exclusion du bloc tubulaire (71).

12. Ensemble pour poser une prothèse d'épaule (1) totale, conforme à l'une quelconque des revendications précédentes.

## Patentansprüche

1. Chirurgische Instrumentenanordnung zum Installieren einer inversen Schulterprothese (1), die umfasst:
- ein Hilfselement (60; 160) zur Formgebung eines Knochenmaterials, das die obere Humerusepiphyse (E) eines Oberarmknochens (H) bildet, in ein einstückiges Volumen (E₃), das sich in der Länge um eine Achse (E_{X-X}) erstreckt, die in Bezug auf die Längsrichtung des Oberarmknochens (H) geneigt ist; und
- ein Hilfselement (70; 170) zum Schneiden der Humerusepiphyse (E), die von dem Formgebungshilfselement (60; 160) geformt ist, um das Volumen des Knochenmaterials (E₃) in einer transversalen Weise zur Achse (E_{X-X}) dieses Volumens zu schneiden, wobei dieses Schneidhilfselement umfasst:
(a) einen rohrförmigen Abschnitt (71), der angepasst ist, auf das Volumen des Knochenmaterials (E₃), das von dem Formgebungshilfselement (60) gestaltet ist, aufgeschoben zu werden, wobei dieser rohrförmige Abschnitt an seinem im Betrieb zum Oberarmknochen (H) gerichteten Längsende einen Querspalt (73) für den Durchgang einer Sägeklinge oder dergleichen begrenzt, um das Volumen des Knochenmaterials (E₃) gemäß einer ersten Ebene (E₄) quer zur Achse (E_{X-X}) dieses Volumens zu schneiden, oder
(b) einen Ringkörper (171), der angepasst ist, um die Humerusepiphyse (E) herum aufgesetzt zu werden, wobei er gleichzeitig das Volumen des Knochenmaterials (E₃), das von dem Formgebungshilfsmittel (160) gestaltet ist, und mindestens ein Teil des Rests der Humerusepiphyse umgibt,
wobei der Ringkörper an seiner im Betrieb zum Oberarmknochen (H) gerichteten Seite eine Fläche (173) zur Führung eines Knochenschneidwerkzeugs begrenzt, um mindestens das Volumen des Knochenmaterials (E₃) in einer quer zu seiner Ache (E_{X-X}) liegenden Weise zu schneiden,
wobei dieser rohrförmige Abschnitt (71) und dieser Ringkörper (171) damit ermöglichen, von dem Oberarmknochen (H) das Volumen des Knochenmaterials (E₃) freizulegen, damit dieses letztere ein Transplantat (2) zur Lateralisation einer Gelenkpfannenkomponente (10) der inversen Schulterprothese (1) in Bezug auf ein Schulterblatt (S) eines mit einer Prothese zu versehenden Patienten bildet.

2. Anordnung nach Anspruch 1, **dadurch gekennzeichnet, dass** sie Mittel (52; 166) zur Resektion des Endes der Humerusepiphyse (E) umfasst, die sich an einem speziellen Resektionshilfselement (50) befinden, das sich von den anderen Hilfselementen (60, 70) der Anordnung unterscheidet, oder die in das Formgebungshilfsmittel integriert sind.

3. Anordnung nach Anspruch 2, **dadurch gekennzeichnet, dass** die Mittel zur Resektion eine flache Fräse (52; 166) aufweisen, derart, dass die Humerusepiphyse (E) gemäß einer zweiten Ebene (E₂) quer zur Achse (E_{X-X}) des Volumens des Knochenmaterials (E₃) entfernt wird.

4. Anordnung nach einem beliebigen der vorhergehenden Ansprüche, **dadurch gekennzeichnet, dass** sie Mittel (53; 153) zur Ausbohrung der Humerusepiphyse (E) umfasst, die angepasst sind, eine Ausnehmung (E₁) in die Humerusepiphyse in auf die Achse (E_{X-X}) des Volumens des Knochenmaterials (E₃) zentrierter Weise einzubringen, und die entweder in dem Formgebungshilfselement oder dem Resektionshilfselement (50) integriert sind oder sich an einem speziellen, zu den anderen Hilfsmitteln (130, 160, 170) der Anordnung unterschiedlichen Bohrhilfselement (167) befinden.

5. Anordnung nach einem beliebigen der vorhergehenden Ansprüche, **dadurch gekennzeichnet, dass** sie außerdem einen Ortsbestimmungsstift (40) oder ein analoges Mittel aufweist, der in die Humerusepiphyse (E) einzuführen ist und angepasst ist, das Formgebungshilfselement (60; 160) und gegebenenfalls mindestens eines der anderen Hilfselemente (50; 167) der Anordnung zu führen.

6. Anordnung nach Anspruch 5, **dadurch gekennzeichnet, dass** sie außerdem ein Hilfselement (30; 130) zum Einführen des Ortsbestimmungsstiftes (40) in die Humerusepiphyse (E) aufweist, das angepasst ist, die Einführungsrichtung dieses Stiftes in Bezug auf den Oberarmknochen (H) einzustellen.

7. Anordnung nach Anspruch 6, **dadurch gekennzeichnet, dass** das Einführungshilfselement (30; 130) einerseits einen Körper (32; 132) in Form einer gewölbten Glocke, die im Inneren so dimensioniert ist, dass sie die obere Humerusepiphyse (E) nach Art einer Kappe bedeckt, und andererseits eine Führung (35) zur Anwendung des Ortsbestimmungsstiftes, der in dem Körper mündet, aufweist.

8. Anordnung nach einem beliebigen der vorhergehenden Ansprüche, **dadurch gekennzeichnet, dass** das Formgebungshilfselement (60; 160) eine Säge (62; 162) in Form einer Glocke aufweist, die angepasst ist, das die Humerusepiphyse (E) bildende Knochenmaterial zu schneiden, wobei sie in das Volumen des Knochenmaterials (E₃) geformt wird.

9. Anordnung nach Anspruch 8, **dadurch gekennzeichnet, dass** die Säge (62; 162) eine zylindrische oder kegelförmige Innenfläche, gegebenenfalls durchbrochen, aufweist, um dem Volumen des Knochenmaterials eine zylindrische (E₃) oder kegelförmige Gesamtform, zentriert auf die Achse (E_{X-X}) dieses Volumens, zu geben.

10. Anordnung nach einem beliebigen der vorhergehenden Ansprüche, **dadurch gekennzeichnet, dass** das Schneidhilfselement (70) den rohrförmigen Abschnitt (71) unter Ausschluss des ringförmigen Körpers (171) aufweist.

11. Anordnung nach einem beliebigen der Ansprüche 1 bis 9, **dadurch gekennzeichnet, dass** das Schneidhilfselement (170) den ringförmigen Körper (171) unter Ausschluss des rohrförmigen Abschnittes (71) aufweist.

12. Anordnung zum in Stellung Bringen einer Gesamtschulterprothese (1) entsprechend einem der vorhergehenden Ansprüche.

## Claims

1. A set of surgical instruments for fitting an inverted shoulder prosthesis (1), which includes:
- an instrument (60; 160) for shaping the bone matter making up the upper humeral epiphysis (E) of a humerus (H), in a one-piece volume (E₃) that extends lengthwise around an axis (E_{X-X}) inclined relative to the longitudinal direction of the humerus (H), and
- an instrument (70; 170) for cutting the humeral epiphysis (E) shaped by the shaping instrument (60; 160), to cut the volume of bone matter (E₃) transversely to the axis (E_{X-X}) of that volume, said cutting instrument including:
(a) a tubular block (71) suitable for being slipped around the volume of the material (E₃) shaped by the shaping instrument (60), that tubular block delimiting, at its longitudinal end turned during use toward the humerus (H), a transverse slot (73) for the passage of a saw blade or similar member, to cut the volume of bone matter (E₃) along a first plane (E₄) transverse to the axis (E_{X-X}) of the volume, or
(b) an annular body (171) suitable for being mounted around the humeral epiphysis (E) while surrounding both the volume of bone matter (E₃) shaped by the shaping instrument (160) and at least a part of the rest of the humeral epiphysis,
the annular body delimiting, at its side turned during use toward the humerus (H), a guide surface (173) for a bone cutting tool, to cut at least the volume of bone matter (E₃) transversely to its axis (E_{X-X}),
this tubular block (71) and this annular body (171) thus making it possible to free the volume of bone matter from the humerus (H) such that it forms a graft (2) for lateralizing a glenoid component (10) of the inverted shoulder prosthesis (1) from the scapula (S) of a patient to be provided with this prosthesis.

2. The set according to claim 1, **characterized in that** it comprises means (52; 166) to resect the end of the humeral epiphysis (E), which are supported by a specific resection instrument (50), separate from the other instruments (60, 70) of the set, or integrated into the shaping instrument (160).

3. The set according to claim 2, **characterized in that** the resecting means includes a flat cutter (52; 166), so as to resect the humeral epiphysis (E) in a second plane (E₂) transverse to the axis (E_{X-X}) of the volume of bone matter (E₃).

4. The set according to any one of the preceding claims, **characterized in that** it comprises means (53; 153) for drilling the humeral epiphysis (E), which are suitable for hollowing out a recess (E₁) in the humeral epiphysis centered on the axis (E_{X-X}) of the volume of the material (E₃) and that are integrated into the shaping instrument or the resection instrument (50), or supported by a specific drilling instrument (167), separate from the other instruments (130, 160, 170) of the set.

5. The set according to any one of the preceding claims, **characterized in that** it further includes a guide pin (40) or similar means, able to be inserted into the humeral epiphysis (E) and suitable for guiding the shaping instrument (60; 160) and optionally at least one of the other instruments (50; 167) of the set.

6. The set according to claim 5, **characterized in that** it further includes an instrument (30; 130) for inserting the guide pin (40) into the humeral epiphysis (E), suitable for adjusting the insertion direction of that pin relative to the humerus (H).

7. The set according to claim 6, **characterized in that** the insertion instrument (30; 130) includes a body (32; 132) in the form of a curved bell on the one hand, inwardly sized to cover the upper humeral epiphysis (E) like a cap, and a guide (35) for applying the guide pin, on the other hand, which emerges in the body.

8. The set according to any one of the preceding claims, **characterized in that** the shaping instrument (60; 160) includes a saw (62; 162) in the shape of a bell, which is suitable for cutting the bone matter making up the humeral epiphysis (E) by shaping it in said volume of bone matter (E₃).

9. The set according to claim 8, **characterized in that** the saw (62; 162) has a cylindrical or frustoconical inner face, optionally openwork, to give the volume of bone matter a cylindrical (E₃) or truncated cone overall shape, centered on the axis (E_{X-X}) of that volume.

10. The set according to any one of the preceding claims, **characterized in that** the cutting instrument (70) includes the tubular block (71), excluding the annular body (171).

11. The set according to any one of claims 1 to 9, **characterized in that** the cutting instrument (70) includes the annular body (171), excluding the tubular block (71).

12. A set for placing a total shoulder prosthesis (1), according to any one of the preceding claims.
